# EUROPEAN PATENT APPLICATION

(11) **EP 4 736 877 A1**
(43) Date of publication of application: **06.05.2026**
(21) Application number: 24941297.4
(22) Date of filing: 02.08.2024
(51) Int. Cl.: A61K 45/06, A61K 39/395, A61P 35/00, A61P 35/02

(54) **PHARMACEUTICAL COMPOSITION AND USE THEREOF**

(30) Priority: 31.05.2024 CN 202410696725
(71) Applicant: The Third Xiangya Hospital of Central South University, Changsha, Hunan 410013 (CN)
(72) Inventor: LV, Ben, Changsha, Hunan 410013 (CN); LI, Yi, Changsha, Hunan 410013 (CN); TANG, Yiting, Changsha, Hunan 410013 (CN)
(74) Representative: impuls legal PartG mbB
(86) International application number: PCT/CN2024/109516
(87) International publication number: WO 2025/246019

(57) **Abstract**

Provided are a pharmaceutical composition and use thereof. The pharmaceutical composition comprises a PD-1 monoclonal antibody or a CTLA-4 monoclonal antibody, and further comprises a Rage inhibitor. The prepared composition, by means of blocking Rage in combination with the PD-1 or CTLA-4 monoclonal antibody, can significantly inhibit tumor growth or even lead to the disappearance of tumors, thereby providing a new research direction for preparing more efficient tumor drugs.

## Description

### TECHNICAL FIELD

The present disclosure belongs to the field of biotechnology, and specifically relates to a pharmaceutical composition and use thereof.

### BACKGROUND

Immune checkpoint inhibitors (ICIs) represent a form of immunotherapy with potentially high specificity and low side effects. They function by modulating immune cell activity and facilitating tumor cell elimination through pathways involving co-inhibitory or co-stimulatory signals. The most well-established ICIs, PD-1/PD-L1 and CTLA-4 monoclonal antibodies, act by releasing inhibitory signals in T lymphocytes. Since the successful introduction of immune checkpoint blockade (ICB) therapy in 2011 for treating unresectable or metastatic melanoma, ICB therapy has provided long-term clinical benefits to patients suffering from various tumor types, including curing a subset of patients. Consequently, the clinical success of ICB therapy has revolutionized the field of cancer immunotherapy. In September 2014, the Food and Drug Administration (FDA) granted accelerated approval to the first PD-1 immune checkpoint inhibitor for second-line treatment of melanoma. To date, numerous ICIs have been approved worldwide for second-/third-line or even first-line treatment of various solid tumors (such as non-small cell lung cancer, urothelial carcinoma, etc.) and for all microsatellite instability-high (MSI-H) solid tumors. As a result, ICB therapy has become a cornerstone of cancer treatment along with conventional treatment methods such as surgery, chemotherapy and radiotherapy.

Although ICB therapy demonstrates significant potential of the human immune system in combating cancer, its efficacy against some "cold" tumors remains limited, and many cancer patients fail to respond to ICB therapy. When used as monotherapy, ICIs achieve response rates ranging from 10% to 35%, with approximately 70% of patients without clinical benefit. Furthermore, resistance to ICB therapy is highly prevalent. Mechanistically, CTLA-4 regulates APC-induced T-cell response by blocking CD28-B7 interaction, whereas PD-1 modulates T cell responses during the effector phase by attenuating TCR signaling. The primary mechanism of PD-1 and CTLA-4 monoclonal antibodies involves releasing the signals that inhibit T cell function to exert antitumor effects. However, they cannot prevent the concurrent progressive exhaustion and exhaustion-induced death of tumor-reactive T cells. Consequently, ICB therapy fails to elicit a lasting antitumor effect, thereby compromising the overall therapeutic outcome.

To achieve optimal clinical outcomes, it is necessary to enhance response rate and duration of the body's immune function. Accordingly, specific small molecule inhibitors hold promise not only for suppressing key oncogenic signaling pathways, but also for sustaining immune cell viability while enhancing their response to exert more long-lasting effect, thereby serving as adjuvant agents to existing ICIs. Current ICB therapy benefits only a minority of cancer patients, and many fail to derive long-term benefits from treatment. Consequently, there is an urgent need in the scientific community to identify a mechanism capable of enhancing immune cell viability and preventing immune cell depletion. This will facilitate the development of ICIs against novel targets, the combination of ICIs targeting different pathways, and the integration of ICIs with other treatment modalities, ultimately improving enhancing immune cell viability and preventing immune cell exhaustion.

### SUMMARY

The present disclosure aims to at least resolve one of the technical problems mentioned above existing in the prior art. To this end, the present disclosure provides a pharmaceutical composition.

The present disclosure further provides a preparation method of the pharmaceutical composition.

The present disclosure further provides use of the pharmaceutical composition.

According to a first aspect of the present disclosure, a pharmaceutical composition is provided, including a PD-1 monoclonal antibody or a CTLA-4 monoclonal antibody, and a receptor for advanced glycation endproducts (RAGE) inhibitor.

In some embodiments of the present disclosure, the RAGE inhibitor includes a molecule capable of blocking transcription or translation of a RAGE gene, or a molecule capable of specifically inhibiting expression or activation of a RAGE protein.

In some embodiments of the present disclosure, the RAGE inhibitor is a nucleic acid molecule, an antibody, or a small-molecule compound.

In some embodiments of the present disclosure, the nucleic acid molecule is selected from the group consisting of siRNA, shRNA, and sgRNA.

In some embodiments of the present disclosure, the small-molecule compound is one or two selected from the group consisting of FPS-ZM1 and Azeliragon. In some embodiments of the present disclosure, the pharmaceutical composition further includes a pharmaceutically acceptable excipient.

In some embodiments of the present disclosure, the pharmaceutically acceptable excipient is one or more selected from the group consisting of a carrier, a diluent, a binder, a lubricant, and a wetting agent.

In some embodiments of the present disclosure, the pharmaceutical composition is in one or more forms selected from the group consisting of a solution, an injection, a spray, a nasal drop, an aerosol, a dry powder inhaler, a tablet, a capsule, and granules.

In some embodiments of the present disclosure, the pharmaceutical composition can be administered into a body such as muscle, intradermal, subcutaneous, venous or mucosal tissues by means of injection, spraying, nasal drops, eye drops, permeation, absorption, or physically or chemically-mediated methods, or is administered into a body after being mixed with or encapsulated by another substance.

According to a second aspect of the present disclosure, there is provided use of the pharmaceutical composition in the preparation of a medicament for treating tumors.

In some embodiments of the present disclosure, the tumor includes at least one of solid tumors and non-solid tumors.

In some embodiments of the present disclosure, the tumor includes at least one of intestinal cancer, gastric cancer, esophageal cancer, hypopharyngeal cancer, laryngeal cancer, oral cancer, nasal cavity cancer, pancreatic cancer, liver cancer, lung cancer, breast cancer, ovarian cancer, cervical cancer, endometrial cancer, vaginal cancer, fallopian tube cancer, kidney cancer, melanoma, brain tumor, thyroid cancer, parathyroid cancer, leukemia, lymphoma, myeloma, sarcoma, prostate cancer, bladder cancer, bile duct cancer, gallbladder cancer, and head and neck squamous cell carcinoma.

In some embodiments of the present disclosure, the intestinal cancer includes at least one of rectal cancer, colorectal cancer, small intestinal cancer and large intestinal cancer.

In some embodiments of the present disclosure, a method of use of the medicament includes: inhibiting or blocking expression of a RAGE target in a subject, and administering a PD-1 monoclonal antibody or a CTLA-4 monoclonal antibody for treatment.

In some embodiments of the present disclosure, inhibiting expression of the RAGE target in the subject includes: administering a RAGE inhibitor.

In some embodiments of the present disclosure, blocking expression of the RAGE target in the subject includes: knocking out the RAGE gene.

In some embodiments of the present disclosure, the subject includes an animal.

In some embodiments of the present disclosure, the animal includes a human and a mouse.

In some embodiments of the present disclosure, when the animal is a mouse, the PD-1 monoclonal antibody is administered at a dose ranging from 8 mg/kg to 12 mg/kg.

In some embodiments of the present disclosure, when the animal is a mouse, the CTLA-4 monoclonal antibody is administered at a dose ranging from 6 mg/kg to 15 mg/kg.

According to some embodiments of the present disclosure, at least the following beneficial effects are achieved: the pharmaceutical composition prepared according to the present disclosure can significantly inhibit tumor growth or even achieves tumor regression by combining a RAGE inhibitor with a PD-1 monoclonal antibody or a CTLA-4 monoclonal antibody, thereby providing a new research direction for developing more efficient antitumor drugs.

### BRIEF DESCRIPTION OF DRAWINGS

The present disclosure is further described below in conjunction with the accompanying drawings and embodiments, in which:

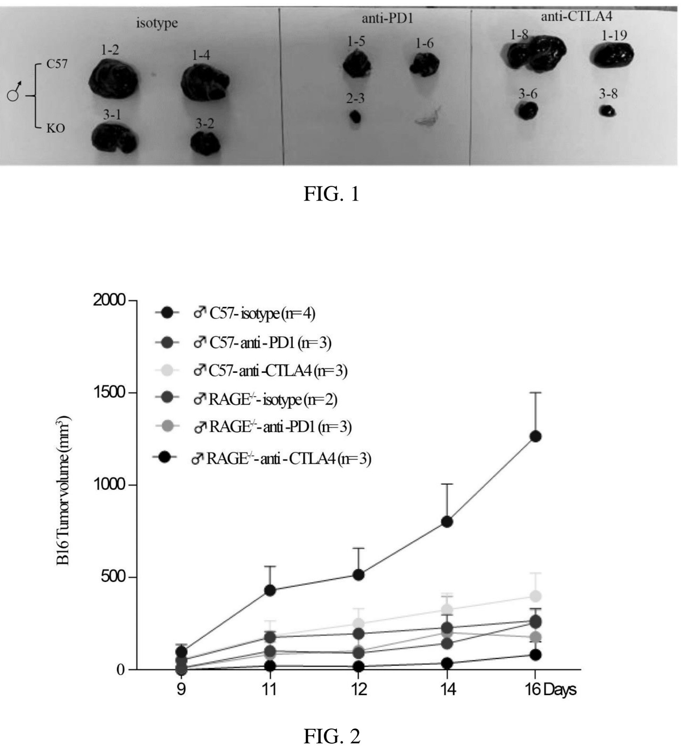

FIG. 1 shows detection results of B16 melanoma in the test example of the present disclosure, wherein numbers 1-2, 1-4, 3-1, 3-2, 1-5, 1-6, 3-3, 1-8, 1-19, 3-6 and 3-8 represent different groups; C57 denotes C57 wild-type mice, KO denotes RAGE gene knockout mice; isotype denotes isotype control IgG; anti-PD-1 denotes PD-1 monoclonal antibody; and anti-CTLA-4 denotes CTLA-4 monoclonal antibody.
FIG. 2 shows tumor size monitoring results for B16 melanoma at different time points in the test example corresponding to the experiment in FIG. 1 of the present disclosure, wherein C57-isotype denotes wild-type mice administered an isotype control IgG; C57-anti PD-1 denotes wild-type mice administered a PD-1 monoclonal antibody; C57-anti CTLA-4 denotes wild-type mice administered a CTLA-4 monoclonal antibody; RAGE⁻/⁻-isotype denotes RAGE gene knockout mice administered an isotype control IgG; RAGE⁻/⁻-anti PD-1 denotes RAGE gene knockout mice administered a PD-1 monoclonal antibody; and RAGE⁻/⁻-anti CTLA-4 denotes RAGE gene knockout mice administered a CTLA-4 monoclonal antibody.

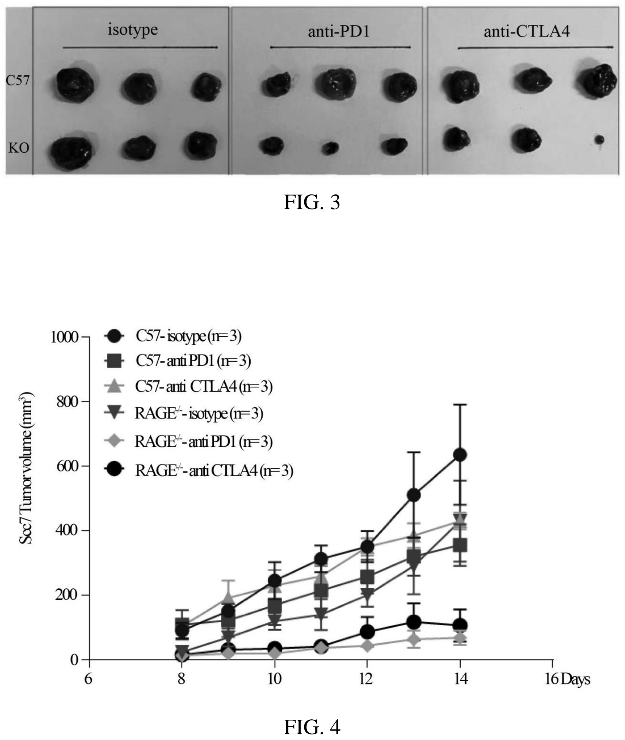

FIG. 3 shows detection results for Scc7 head and neck squamous cell carcinoma in the test example of the present disclosure, where C57 denotes C57 wild-type mice; KO denotes RAGE gene knockout mice; isotype denotes an isotype control IgG; anti-PD-1 denotes a PD-1 monoclonal antibody; and anti-CTLA-4 denotes a CTLA-4 monoclonal antibody.
FIG. 4 shows tumor size monitoring results for the Scc7 head and neck squamous cell carcinoma cells at different time points in the test example corresponding to the experiment in FIG. 3 of the present disclosure, wherein C57-isotype denotes wild-type mice administered a isotype control IgG; C57-anti PD-1 denotes wild-type mice administered a PD-1 monoclonal antibody; C57-anti CTLA-4 denotes wild-type mice administered a CTLA-4 monoclonal antibody; RAGE⁻/⁻-isotype denotes RAGE gene knockout mice administered an isotype control IgG; RAGE⁻/⁻-anti PD-1 denotes RAGE gene knockout mice administered a PD-1 monoclonal antibody; and RAGE⁻/⁻-anti CTLA-4 denotes RAGE gene knockout mice administered a CTLA-4 monoclonal antibody.

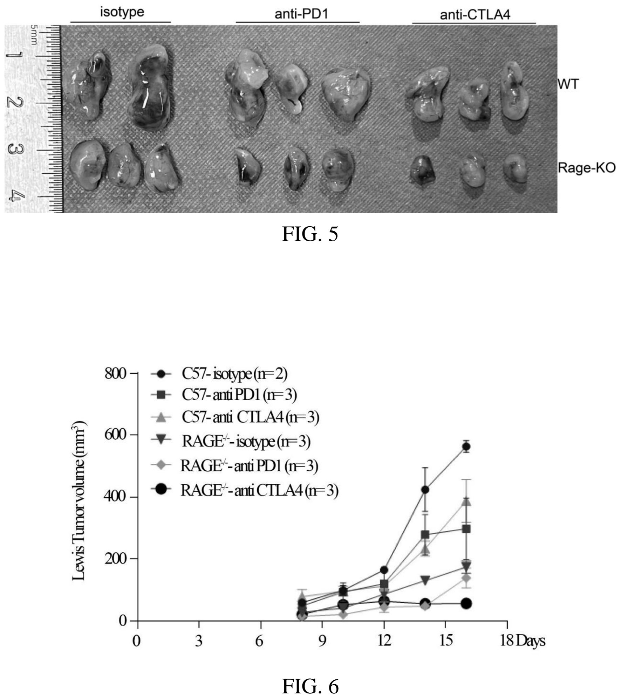

FIG. 5 shows detection results for Lewis pulmonary adenocarcinoma in the test example of the present disclosure, wherein WT denotes C57 wild-type mice; RAGE-KO denotes RAGE gene knockout mice; isotype denotes an isotype control IgG; anti-PD-1 denotes a PD-1 monoclonal antibody; and anti-CTLA-4 denotes a CTLA-4 monoclonal antibody.
FIG. 6 shows tumor size monitoring results for Lewis pulmonary adenocarcinoma cells at different time points in the test example corresponding to the experiment in FIG. 5 of the present disclosure, wherein C57-isotype denotes wild-type mice administered an isotype control IgG; C57-anti PD-1 denotes wild-type mice administered a PD-1 monoclonal antibody, C57-anti CTLA-4 denotes wild-type mice administered a CTLA-4 monoclonal antibody, RAGE⁻/⁻-isotype denotes RAGE gene knockout mice administered an isotype control IgG, RAGE⁻/⁻-anti PD-1 denotes RAGE gene knockout mice administered a PD-1 monoclonal antibody, and RAGE⁻/⁻-anti CTLA-4 denotes RAGE gene knockout mice administered a CTLA-4 monoclonal antibody.

### DETAILED DESCRIPTION

The concept and technical effects of the present disclosure will be clearly and completely described below in conjunction with the embodiments, so as to enable a full understanding of the purpose, features, and effects of the present disclosure. Obviously, the described examples are only a part of the examples of the present disclosure, rather than all the examples. Based on the examples of the present disclosure, other examples obtained by those skilled in the art without creative efforts shall all fall within the protection scope of the present disclosure.

Experimental Materials: 6-8-week-old C57 mice purchased from Hunan Slike Jingda Laboratory Animal Co., Ltd., China; wherein RAGE^{-/-} refers to RAGE gene knockout mice on a C57 background, and the gene knockout mice and their littermate control mice were 8-12-week-old and were generously provided by Southern Medical University. Detailed methods for the generation and identification of the mice are described in the reference *"RAGE Control of Diabetic Nephropathy in a Mouse Model: Effects of RAGE Gene Disruption and Administration of Low-Molecular Weight Heparin".*

### Example 1

This example provided a pharmaceutical composition. The pharmaceutical composition included a RAGE inhibitor and a PD-1 monoclonal antibody.

### Example 2

This example provided a pharmaceutical composition. The pharmaceutical composition included a RAGE inhibitor and a CTLA-4 monoclonal antibody.

### Test example

### 1. Use of the pharmaceutical composition in treatment of B16 melanoma.

### Experimental method:

Animal grouping: WT-isotype group (wild-type mice administered an isotype control IgG, C57-isotype); WT-anti PD-1 group (wild-type mice administered a PD-1 monoclonal antibody, C57-anti PD-1); WT-anti CTLA-4 group (wild-type mice administered a CTLA-4 monoclonal antibody, C57-anti CTLA-4); RAGE⁻/⁻-isotype group (RAGE gene knockout mice administered an isotype control IgG); RAGE⁻/⁻-anti PD-1 group (RAGE gene knockout mice administered a PD-1 monoclonal antibody); and RAGE⁻/⁻-anti CTLA-4 group (RAGE gene knockout C57 mice administered a CTLA-4 monoclonal antibody). For each experiment, each group used at least 2 mice (3 mice in each group in this example).

Specific experiment included:
(1) shaving a left dorsal area of the mice in all groups one day in advance;
(2) dissociating B16 melanoma cells: washing the B16 melanoma cells with pre-cooled DPBS, and centrifuging at 4 °C and 800 rpm for 5 min to remove a supernatant; and repeating the washing for 3 times;
(3) counting cells: adjusting the B16 melanoma cells to a concentration of 1 × 10⁶ cells per 100 µL of DPBS;
(4) subcutaneously injecting 1 × 10⁶ B16 melanoma cells into a left dorsal flank of each mouse within 1 h after cell collection;
(5) intraperitoneally injecting 10 mg/kg of PD-1 monoclonal antibody to the WT-anti PD-1 group and the RAGE⁻/⁻-anti PD-1 group respectively on the day of and day 2 after subcutaneous injection of B16 melanoma cells, and intraperitoneally injecting 10 mg/kg of isotype control IgG to the corresponding WT-isotype group and RAGE⁻/⁻-isotype group respectively;
(6) intraperitoneally injecting 8 mg/kg of CTLA-4 monoclonal antibody to the WT-anti CTLA-4 group and the RAGE⁻/⁻-anti CTLA-4 group respectively on day 3 after subcutaneous injection of B16 melanoma cells, and intraperitoneally injecting 8 mg/kg of isotype control IgG to the corresponding WT-isotype group and RAGE⁻/⁻-isotype group respectively;
(7) intraperitoneally injecting 4 mg/kg of CTLA-4 monoclonal antibody to the WT-anti CTLA-4 group and the RAGE⁻/⁻-anti CTLA-4 group respectively on day 6 and day 9 after subcutaneous injection of B16 melanoma cells, and intraperitoneally injecting 4 mg/kg of isotype control IgG to the corresponding WT-isotype group and RAGE⁻/⁻-isotype group respectively; and
(8) at day 14 after modeling, sampling for detection.

The experimental results are shown in FIGS. 1-2. As can be seen, the pharmaceutical composition of the present disclosure can significantly inhibit growth of B16 melanoma or even achieves tumor regression.

### 2. Use of the pharmaceutical composition in treatment of Scc7 head and neck squamous cell carcinoma

### Experimental method:

Animal grouping: WT-isotype group, WT-anti PD-1 group, WT-anti CTLA-4 group, RAGE⁻/⁻-isotype group, RAGE⁻/⁻-anti PD-1 group, RAGE⁻/⁻-anti CTLA-4 group. For each experiment, each group used at least 2 mice (3 mice in each group in this example).

Specific experiment included:
(1) shaving a left dorsal area of the mice in all groups one day in advance;
(2) dissociating Scc7 head and neck squamous cell carcinoma cells: washing the Scc7 head and neck squamous cell carcinoma cells with pre-cooled DPBS, and centrifuging at 4 °C and 800 rpm for 5 min to remove a supernatant; and repeating the washing for 3 times;
(3) counting cells: adjusting the Scc7 cells to a concentration of 1×10⁶ cells per 100 µL of DPBS;
(4) subcutaneously injecting 1×10⁶ Scc7 head and neck squamous cell carcinoma cells into a left dorsal flank of each mouse within 1 h after cell collection;
(5) intraperitoneally injecting 10 mg/kg of PD-1 monoclonal antibody to the WT-anti PD-1 group and the RAGE⁻/⁻-anti PD-1 group respectively on the day of and day 2 after subcutaneous injection of Scc7 cells, and intraperitoneally injecting 10 mg/kg of isotype control IgG to the corresponding WT-isotype group and RAGE⁻/⁻-isotype group respectively;
(6) intraperitoneally injecting 8 mg/kg of CTLA-4 monoclonal antibody to the WT-anti CTLA-4 group and the RAGE⁻/⁻-anti CTLA-4 group respectively on day 3 after subcutaneous injection of Scc7 cells, and intraperitoneally injecting 8 mg/kg of isotype control IgG to the corresponding WT-isotype group and RAGE⁻/⁻-isotype group respectively;
(7) intraperitoneally injecting 4 mg/kg of CTLA-4 monoclonal antibody to the WT-anti CTLA-4 group and the RAGE⁻/⁻-anti CTLA-4 group respectively on day 6 and day 9 after subcutaneous injection of Scc7 cells, and intraperitoneally injecting 4 mg/kg isotype control IgG to the corresponding WT-isotype group and RAGE⁻/⁻-isotype group respectively; and
(8) at day 14 after modeling, sampling for detection.

The experimental results are shown in FIGS. 3-4. As can be seen, the pharmaceutical composition of the present disclosure can significantly inhibit growth of Scc7 head and neck squamous cell carcinoma.

### 3. Use of the pharmaceutical composition in treatment of Lewis pulmonary adenocarcinoma.

### Experimental method:

Animal grouping: WT-isotype group, WT-anti PD-1 group, WT-anti CTLA-4 group, RAGE⁻/⁻-isotype group, RAGE⁻/⁻-anti PD-1 group, RAGE⁻/⁻-anti CTLA-4 group. For each experiment, each group used at least 2 mice (3 mice in each group in this example).

Specific experiment included:
(1) shaving a left dorsal area of the mice in all groups one day in advance;
(2) dissociating Lewis pulmonary adenocarcinoma cells: washing the Lewis pulmonary adenocarcinoma cells with pre-cooled DPBS, and centrifuging at 4 °C and 800 rpm for 5 min to remove a supernatant; and repeating the washing for 3 times;
(3) counting cells: adjusting the cells to a concentration of 1 × 10⁶ cells per 100 µL of DPBS;
(4) subcutaneously injecting 1 × 10⁶ Lewis pulmonary adenocarcinoma cells into a left dorsal flank of each mouse within 1 h of cell collection;
(5) intraperitoneally injecting 10 mg/kg of PD-1 monoclonal antibody to the WT-anti PD-1 group and the RAGE⁻/⁻-anti PD-1 group respectively on the day of and day 2 after subcutaneous injection of Lewis pulmonary adenocarcinoma cells, and intraperitoneally injecting 10 mg/kg isotype control IgG to the corresponding WT-isotype group and RAGE⁻/⁻-isotype group respectively;
(6) intraperitoneally injecting 8 mg/kg of CTLA-4 monoclonal antibody to the WT-anti CTLA-4 group and the RAGE⁻/⁻-anti CTLA-4 group respectively on day 3 after subcutaneous injection of Lewis pulmonary adenocarcinoma cells, and intraperitoneally injecting 8 mg/kg of isotype control IgG to the corresponding WT-isotype group and RAGE⁻/⁻-isotype group respectively;
(7) intraperitoneally injecting 4 mg/kg of CTLA-4 monoclonal antibody to the WT-anti CTLA-4 group and the RAGE⁻/⁻-anti CTLA-4 group respectively on day 6 and day 9 after subcutaneous injection of Lewis pulmonary adenocarcinoma cells, and intraperitoneally injecting 4 mg/kg of isotype control IgG to the corresponding WT-isotype group and RAGE⁻/⁻-isotype group respectively; and
(8) at day 14 after modeling, sampling for detection.

The experimental results are shown in FIGS. 5-6. As can be seen, the pharmaceutical composition of the present disclosure can significantly inhibit growth of Lewis pulmonary adenocarcinoma.

The examples of the present disclosure have been described in detail above with reference to the accompanying drawings. However, the present disclosure is not limited to the above-described examples, and various modifications may be made without departing from the protection scope of the present disclosure within the scope of knowledge possessed by those having ordinary skills in the art. In addition, the examples of the present disclosure and the features in the examples may be combined with each other without conflict.

## Claims

1. A pharmaceutical composition, comprising a PD-1 monoclonal antibody or a CTLA-4 monoclonal antibody, and a RAGE inhibitor.

2. The pharmaceutical composition according to claim 1, wherein the RAGE inhibitor comprises a molecule capable of blocking transcription or translation of a RAGE gene, or a molecule capable of specifically inhibiting expression or activation of a RAGE protein.

3. The pharmaceutical composition according to claim 2, wherein the RAGE inhibitor is a nucleic acid molecule, an antibody, or a small-molecule compound.

4. The pharmaceutical composition according to claim 3, wherein the nucleic acid molecule is selected from the group consisting of siRNA, shRNA, and sgRNA; and/or, the small-molecule compound is one or two selected from the group consisting of FPS-ZM1 and Azeliragon.

5. The pharmaceutical composition according to claim 1, further comprising a pharmaceutically acceptable excipient; preferably, the pharmaceutically acceptable excipient comprises at least one of a diluent, a vehicle, a filler, a binder, a disintegrant, an absorption promoter, a surfactant, an adsorbent carrier, a lubricant, a sweetener, and a flavoring agent.

6. The pharmaceutical composition according to claim 1, wherein the pharmaceutical composition is in one or more forms selected from the group consisting of a solution, an injection, a spray, a nasal drop, an aerosol, a dry powder inhaler, a tablet, a capsule, and granules.

7. The pharmaceutical composition according to claim 1, wherein the pharmaceutical composition can be administered into a body such as muscle, intradermal, subcutaneous, venous or mucosal tissues by means of injection, spraying, nasal drops, eye drops, permeation, absorption, or physically or chemically-mediated methods, or is administered into a body after being mixed with or encapsulated by another substance.

8. Use of the pharmaceutical composition according to any one of claims 1 to 7 in the preparation of a medicament for treating tumors.

9. The use according to claim 8, wherein the tumor comprises at least one of solid tumors and non-solid tumors; preferably, the tumor comprises at least one of intestinal cancer, gastric cancer, esophageal cancer, hypopharyngeal cancer, laryngeal cancer, oral cancer, nasal cavity cancer, pancreatic cancer, liver cancer, lung cancer, breast cancer, ovarian cancer, cervical cancer, endometrial cancer, vaginal cancer, fallopian tube cancer, kidney cancer, melanoma, brain tumor, thyroid cancer, parathyroid cancer, leukemia, lymphoma, myeloma, sarcoma, prostate cancer, bladder cancer, bile duct cancer, gallbladder cancer, and head and neck squamous cell carcinoma; more preferably, the intestinal cancer comprises at least one of rectal cancer, colorectal cancer, small intestinal cancer and large intestinal cancer.

10. The use according to claim 8, wherein a method of use of the medicament comprises: inhibiting or blocking expression of a RAGE target in a subject, and administering a PD-1 monoclonal antibody or a CTLA-4 monoclonal antibody for treatment.
